# EUROPÄISCHE PATENTSCHRIFT

(11) **EP 0 736 080 B1**
(45) Veröffentlichungstag und Bekanntmachung des Hinweises auf die Patenterteilung: **31.03.1999**
(21) Anmeldenummer: 95902137.9
(22) Anmeldetag: 09.12.1994
(51) Int. Cl.: C10L 1/00, C10M 171/00, C10L 1/22, C09B 31/053, C09B 29/095, C09B 29/08

(54) **VERWENDUNG VON AZOFARBSTOFFEN ZUM MARKIEREN VON KOHLENWASSERSTOFFEN SOWIE NEUE AZOFARBSTOFFE**
USE OF AZO DYES FOR MARKING HYDROCARBONS AND NOVEL AZO DYES
UTILISATION DE COLORANTS AZOIQUES POUR LE MARQUAGE D'HYDROCARBURES ET NOUVEAUX COLORANTS AZOIQUES

(30) Priorität: 22.12.1993 DE 4343823
(43) Veröffentlichungstag der Anmeldung: 09.10.1996
(73) Patentinhaber: BASF Aktiengesellschaft, 67063 Ludwigshafen (DE)
(72) Erfinder: ZEIDLER, Georg, D-67125 Dannstadt-Schauernheim (DE); SCHOLZ, Gerhard, D-67059 Ludwigshafen (DE); KRÄH, Claudia, D-67112 Mutterstadt (DE); BECK, Karin, Heidrun, D-67067 Ludwigshafen (DE); MAYER, Udo, D-67227 Frankenthal (DE)
(86) Internationale Anmeldenummer: EP9404091
(87) Internationale Veröffentlichungsnummer: WO9517483

(56) Entgegenhaltungen:
- EP-A- 0 256 460
- EP-A- 0 446 731
- EP-A- 0 499 845
- EP-A- 0 519 270
- EP-A- 0 553 858
- DE-A- 3 700 329
- FR-A- 2 232 636
- FR-A- 2 254 610
- FR-A- 2 342 168
- GB-A- 953 719
- GB-A- 1 153 144
- US-A- 3 218 309

## Beschreibung

Die vorliegende Erfindung betrifft die Verwendung von Azofarbstoffen der Formel I 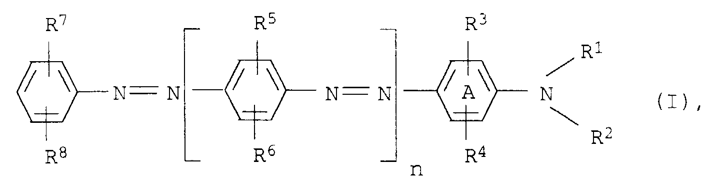 in der
der Ring A benzoanelliert sein kann,
- n: 0 oder 1,
- R¹: Wasserstoff oder C₁-C₁₅-Alkyl, das durch 1 bis 4 Sauerstoffatome in Etherfunktion unterbrochen sein kann,
- R²: C₁-C₁₅-Alkyl, das durch 1 bis 4 Sauerstoffatome in Etherfunktion unterbrochen sein kann, oder einen Rest der Formel L-NX¹X², worin L für C₂-C₈-Alkylen und X¹ und X² unabhängig voneinander jeweils für C₁-C₆-Alkyl oder zusammen mit dem sie verbindenden Stickstoffatom für einen 5- oder 6-gliedrigen gesättigten heterocyclischen Rest, der noch ein Sauerstoffatom im Ring enthalten kann, stehen,
- R³, R⁴, R⁵, R⁶ und R⁷: unabhängig voneinander jeweils Wasserstoff, C₁-C₁₅-Alkyl oder C₁-C₁₅-Alkoxy und
- R⁸: Wasserstoff, C₁-C₁₅-Alkyl, C₁-C₁₅-Alkoxy, Cyano, Nitro oder einen Rest der Formel COOX³, worin X³ für Wasserstoff, C₁-C₁₅-Alkyl, das durch 1 bis 4 Sauerstoffatome in Etherfunktion unterbrochen sein kann, oder für einen Rest der Formel L-NX¹X² steht, worin L, X¹ und X² jeweils die obengenannten Bedeutung besitzen, bedeuten,
mit der Maßgabe, daß die Farbstoffe der Formel I keine Acetalgruppe im Molekül aufweisen,
als pH-Wert-abhängige Markierungsmittel für Kohlenwasserstoffe, wobei die Azofarbstoffe der Formel I in solcher Konzentration den Kohlenwasserstoffen zugesetzt werden, daß die Kohlenwasserstoffe dadurch für das menschliche Auge entweder überhaupt nicht oder nur wenig sichtbar angefärbt sind, sie jedoch unter Einwirkung einer Protonsäure, gegebenenfalls in Anwesenheit eines Halogenids der Metalle Zink, Aluminium oder Zinn, eine Farbreaktion ergeben,
ein Verfahren zum Nachweis dieser Azofarbstoffe in Kohlenwasserstoffen sowie neue Azofarbstoffe.

Aus der US-A 5 145 573, US-A-5 182 372 sowie der EP-A-499 845 sind bereits Azofarbstoffe bekannt, die als Markierungsmittel für Mineralöle dienen. Es hat sich jedoch gezeigt, daß die dort beschriebenen Farbstoffe eine ungenügende Verdünnbarkeit in Kohlenwasserstoffen aufweisen.

In der US-A-4 009 008 wird weiterhin ein Verfahren zum Markieren von Mineralölen mittels Disazofarbstoffen beschrieben, bei dem man den dem Mineralöl zugesetzten Farbstoff sichtbar macht, indem man dem markierten Mineralöl ein Adsorptionsmittel zusetzt, das andere farbige Bestandteile des Mineralöls bindet.

Aufgabe der vorliegenden Erfindung war es, neue Mittel zum Markieren von Kohlenwasserstoffen bereitzustellen. Die neuen Mittel sollten leicht zugänglich und gut in Kohlenwasserstoffen löslich sein. Außerdem sollten sie in einfacher Weise nachgewiesen werden können. Dabei sollten selbst noch sehr kleine Mengen an Markierstoff durch eine starke Farbreaktion sichtbar gemacht werden können.

Demgemäß wurde gefunden, daß sich die eingangs näher bezeichneten Azofarbstoffe der Formel I vorteilhaft als pH-Wert-abhängige Markierungsmittel für Kohlenwasserstoffe eignen.

Alle in den hier genannten Formeln auftretenden Alkyl- und Alkylenreste können sowohl geradkettig als auch verzweigt sein.

Wenn X¹ und X² zusammen mit dem sie verbindenden Stickstoffatom einen 5- oder 6-gliedrigen gesättigten heterocyclischen Rest, der noch ein Sauerstoffatom im Ring enthalten kann, bedeuten, so können dafür z.B. Pyrrolidinyl, Piperidinyl oder Morpholinyl in Betracht kommen.

Reste R¹, R², R³, R⁴, R⁵, R⁶, R⁷, R⁸, X¹, X² und X³ sind z.B. Methyl, Ethyl, Propyl, Isopropyl, Butyl, Isobutyl, sec-Butyl, Pentyl, Isopentyl, Neopentyl, tert-Pentyl, Hexyl oder 2-Methylpentyl.

Reste R¹, R², R³, R⁴, R⁵, R⁶, R⁷, R⁸ und X³ sind weiterhin z.B. Heptyl, Octyl, 2-Ethylhexyl, Isooctyl, Nonyl, Isononyl, Decyl, Isodecyl, Undecyl, Dodecyl, Tridecyl, 3,5,5,7-Tetramethylnonyl, Isotridecyl, Tetradecyl oder Pentadecyl (die obigen Bezeichnungen Isooctyl, Isononyl, Isodecyl und Isotridecyl sind Trivialbezeichnungen und stammen von den nach der Oxosynthese erhaltenen Alkoholen - vgl. dazu Ullmann's Encyclopedia of Industrial Chemistry, 5th Edition, Vol. A1, Seiten 290 bis 293, sowie Vol. A 10, Seiten 284 und 285).

Reste R³, R⁴, R⁵, R^{6,} R⁷ und R⁸ sind weiterhin z.B. Methoxy, Ethoxy, Propoxy, Isopropoxy, Butoxy, Isobutoxy, sec-Butoxy, Pentyloxy, Isopentyloxy, Neopentyloxy, tert-Pentyloxy, Hexyloxy, 2-Methylpentyloxy, Heptyloxy, Octyloxy, 2-Ethylhexyloxy, Isooctyloxy, Nonyloxy, Isononyloxy, Decyloxy, Isodecyloxy, Undecyloxy, Dodecyloxy, Tridecyloxy, 3,5,5,7-Tetramethylnonyloxy, Isotridecyloxy, Tetradecyloxy oder Pentadecyloxy.

Reste L sind z.B. (CH₂)₂, (CH₂)₃, (CH₂)₄, (CH₂)₅, (CH₂)₆, (CH₂)₇, (CH₂)₈, CH(CH₃)CH₂ oder CH(CH₃)CH(CH₃).

Reste R¹, R² und X³ sind weiterhin z.B. 2-Methoxyethyl, 2-Ethoxyethyl, 2-Propoxyethyl, 2-Isopropoxyethyl, 2-Butoxyethyl, 2- oder 3-Methoxypropyl, 2- oder 3-Ethoxypropyl, 2- oder 3-Propoxypropyl, 2- oder 3-Butoxypropyl, 2- oder 4-Methoxybutyl, 2- oder 4-Ethoxybutyl, 2- oder 4-Propoxybutyl, 3,6-Dioxaheptyl, 3,6-Dioxyoctyl, 4,8-Dioxanonyl, 3,7-Dioxaoctyl, 3,7-Dioxanonyl, 4,7-Dioxaoctyl, 4,7-Dioxanonyl, 2- oder 4-Butoxybutyl, 4,8-Dioxadecyl, 4,7-Di-oxaundecyl, 3,6,9-Trioxadecyl, 3,6,9-Trioxaundecyl, 3,6,9-Trioxadodecyl, 4,7,10-Trioxaundecyl, 3,6,9,12-Tetraoxatridecyl oder 3,6,9,12-Tetraoxatetradecyl.

Bevorzugt werden Azofarbstoffe der Formel Ia 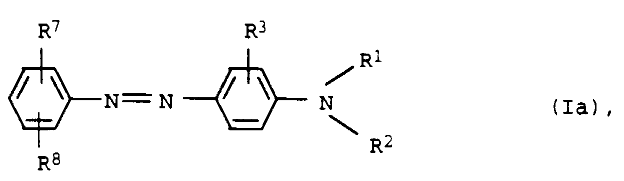 in der
- R¹: Wasserstoff oder C₁-C₁₅-Alkyl, und
- R² und R³: unabhängig voneinander jeweils C₁-C₁₅-Alkyl bedeuten und
- R⁷ und R⁸: jeweils die obengenannte Bedeutung besitzen,
zum Markieren von Kohlenwasserstoffen verwendet.

Weiterhin bevorzugt werden Azofarbstoffe der Formel Ib 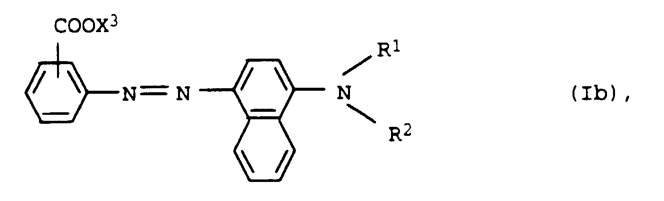 in der
- R¹: Wasserstoff oder C₁-C₁₅-Alkyl und
- R²: C₁-C₁₅-Alkyl bedeuten und
- X³: die obengenannte Bedeutung besitzt,
zum Markieren von Kohlenwasserstoffen verwendet.

Weiterhin bevorzugt werden Azofarbstoffe der Formel Ic 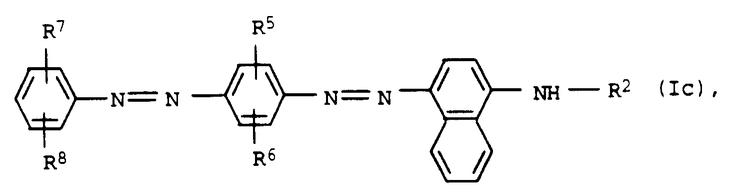 in der
R² C₁-C₁₅-Alkyl bedeutet und
R⁵, R⁶, R⁷ und R⁸ jeweils die obengenannte Bedeutung besitzen, zum Markieren von Kohlenwasserstoffen verwendet.

Besonders bevorzugt werden Azofarbstoffe der Formel Ia, in der R¹ und R² unabhängig voneinander jeweils C₁-C₁₃-Alkyl, R³ Methyl, R⁷ Wasserstoff und R⁸ einen Rest der Formel CCOX³ bedeuten, worin X³ für C₁-C₁₃-Alkyl steht, zum Markieren von Kohlenwasserstoffen verwendet.

Besonders bevorzugt werden weiterhin Azofarbstoffe der Formel Ic, in der R² C₁-C₁₃-Alkyl, R⁵ und R⁷ jeweils Methyl und R⁶ und R⁸ jeweils Wasserstoff bedeuten, zum Markieren von Kohlenwasserstoffen verwendet.

Einige der Farbstoffe der Formel I sind bekannt und z.B. in der GB-A-953 719, US-A-3 218 309 oder US-A-4 037 007 beschrieben.

Als pH-Wert-abhängige Markierungsmittel im erfindungsgemäßen Sinn sind solche Azofarbstoffe der Formel I zu verstehen, die unter Einwirkung einer Protonsäure, gegebenenfalls in Anwesenheit eines Halogenids der Metalle Zink, Aluminium oder Zinn, eine Farbreaktion, d.h. einen Farbumschlag, einhergehend mit einer Farbvertiefung, ergeben.

Unter Markierung im erfindungsgemäßen Sinn ist ein Zusatz der Azofarbstoffe der Formel I in solcher Konzentration zu Kohlenwasserstoffen zu verstehen, daß die Kohlenwasserstoffe dadurch für das menschliche Auge entweder überhaupt nicht oder nur wenig sichtbar angefärbt sind, wobei jedoch die Farbstoffe der Formel I durch die hier näher beschriebenen Nachweismethoden leicht und deutlich sichtbar detektierbar sind.

Unter Kohlenwasserstoffen im erfindungsgemäßen Sinn sind aliphatische oder aromatische Kohlenwasserstoffe zu verstehen, die unter Normalbedingungen in flüssigem Aggregatzustand vorliegen. Dies sind Insbesondere Mineralöle, beispielsweise Treibstoffe, wie Benzin, Kerosin oder Dieselöl, oder Öle, wie Heizöl oder Motorenöl.

Die Azofarbstoffe der Formel I eignen sich insbesondere zum Markieren von Mineralölen, bei denen eine Kennzeichnung gefordert wird, z.B. aus steuerlichen Gründen. Um die Kosten der Kennzeichnung gering zu halten, strebt man dabei an, für die Markierung möglichst geringe Mengen an Markierungsmittel anzuwenden.

Zum Markieren von Kohlenwasserstoffen werden die Azofarbstoffe der Formel I entweder in Substanz oder in Form von Lösungen angewandt. Als Lösungsmittel eignen sich organische Lösungsmittel. Vorzugsweise kommen aromatische Kohlenwasserstoffe, wie Toluol, Xylol, Dodecylbenzol, Diisopropylnaphthalin oder ein Gemisch höherer Aromaten, das unter dem Namen Shellsol® AB (Fa. Shell) handelsüblich ist, zur Anwendung. Um eine hohe Viskosität der resultierenden Lösungen zu vermeiden, wählt man im allgemeinen eine Konzentration an Azofarbstoff I von 20 bis 80 Gew.-%, bezogen auf die Lösung.

Zur Verbesserung der Löslichkeit können auch noch weitere Cosolventien, z.B. Alkohole, wie Methanol, Ethanol, Propanol, Isopropanol, Butanol, Isobutanol, Pentanol, Hexanol, Heptanol, Octanol, 2-Ethylhexanol oder Cyclohexanol, Glykole, wie Butylethylenglykol oder Methylpropylenglykol, Amine, wie Triethylamin, Diisooctylamin, Dicyclohexylamin, Anilin, N-Methylanilin, N,N-Dimethylanilin, Toluidin oder Xylidin, Alkanolamine, wie 3-(2-Methoxyethoxy)propylamin, o-Kresol, m-Kresol, p-Kresol, Ketone, wie Diethylketon oder Cyclohexanon, Lactame, wie γ-Butyrolactam, Carbonate, wie Ethylencarbonat oder Propylencarbonat, Phenole, wie t-Butylphenol oder Nonylphenol, Ester, wie Phthalsäuremethylester, Phthalsäureethylester, Phthalsäure-(2-ethylhexyl)ester, Essigsäureethylester, Essigsäurebutylester oder Essigsäurecyclohexylester, Amide, wie N,N-Dimethylformamid, N,N-Diethylacetamid oder N-Methylpyrrolidon, oder deren Mischungen verwendet werden.

Mittels den erfindungsgemäß anzuwendenden Azofarbstoffen der Formel I gelingt es sehr einfach, markierte Kohlenwasserstoffe nachzuweisen, selbst wenn die Markierungssubstanzen nur in einer Konzentration von ungefähr 10 ppm oder darunter vorliegen.

In manchen Fällen ist auch vorteilhaft, Mischungen von Farbstoffen der Formel I untereinander als Markierungssubstanzen zu verwenden.

Der Nachweis der Anwesenheit der als Markierungsstoffe angewandten Azofarbstoffe der Formel I in Kohlenwasserstoffen gelingt vorteilhaft, wenn man den Kohlenwasserstoff mit einem wäßrig-alkoholischen oder alkoholischem Medium behandelt, das eine Protonsäure und gegebenenfalls ein Halogenid der Metalle Zink, Aluminium oder Zinn enthält. Bei der Verwendung von wäßrig-alkoholischen Medien beträgt das Gewichtsverhältnis Wasser : Alkohol 0,5 : 1 bis 4 : 1, vorzugsweise ca. 1:1.

Bei Zusatz der Protonsäure und gegebenenfalls des Metallhalogenids zum markierten Kohlenwasserstoff resultiert eine deutlich sichtbare Farbreaktion und der Azofarbstoff I tritt, bei Anwendung eines wäßrig-alkoholischen Mediums, in die wäßrig-alkoholische Phase über.

Geeignete Alkohole sind z.B. Ethanol, Propanol, Isopropanol, 1-Methoxypropan-2-ol, Ethylenglykol oder 1,2- oder 1,3-Propylenglykol. Die Verwendung von Ethanol ist bevorzugt.

Geeignete Protonsäuren für das erfindungsgemäße Verfahren sind insbesondere sogenannte starke Säuren, d.h. Protonsäuren deren pKa-Wert ≤ 3,5 ist. Als solche Säuren kommen beispielsweise anorganische oder organische Säuren, wie Perchlorsäure, Iodwasserstoffsäure, Chlorwasserstoffsäure, Bromwasserstoffsäure, Flußsäure, Schwefelsäure, Salpetersäure, Phosphorsäure, Benzolsulfonsäure, Toluolsulfonsäure, Naphthalinsulfonsäure, Methansulfonsäure, Oxalsäure, Maleinsäure, Chloressigsäure, Dichloressigsäure oder Bromessigsäure in Betracht. In manchen Fällen kann es von Vorteil sein, diese Säuren, z.B. durch Zugabe von Essigsäure, abzupuffern.

Neben o- oder p-Toluolsulfonsäure sind besonders anorganische Säuren hervorzuheben, wobei Salzsäure oder Schwefelsäure besondere Bedeutung zukommt.

Geeignete Halogenide der Metalle Zink, Aluminium oder Zinn sind z.B. Zinkchlorid, Zinkbromid, Aluminiumchlorid, Aluminiumbromid oder Zinntetrachlorid. Besonders hervorzuheben ist Zinkchlorid.

Es genügt in der Regel, eine Menge von ungefähr 10 bis 50 ml des erfindungsgemäß markierten Kohlenwasserstoffs mit 10 bis 50 ml einer wäßrig-alkoholischen oder alkoholischen Lösung einer Protonsäure, gegebenenfalls unter Zusatz des Metallhalogenids, auszuschütteln, um diese Farbreaktion zu erhalten. Es ist auch möglich, eine wäßrig-alkoholische Lösung des Metallhalogenids alleine zu benutzen, da diese ebenfalls sauer reagiert.

Die Konzentration der Protonsäure in der wäßrig-alkoholischen oder alkoholischen Lösung beträgt dabei in der Regel 5 bis 50 Gew.-%, vorzugsweise 10 bis 30 Gew.-%. Die Konzentration an Metallhalogenid liegt im allgemeinen bei 0 bis 50 Gew.-%, vorzugsweise bei 5 bis 20 Gew.-%, jeweils bezogen auf das Gewicht der Lösung.

Ein weiterer Gegenstand der vorliegenden Erfindung sind Azofarbstoffe der Formel II 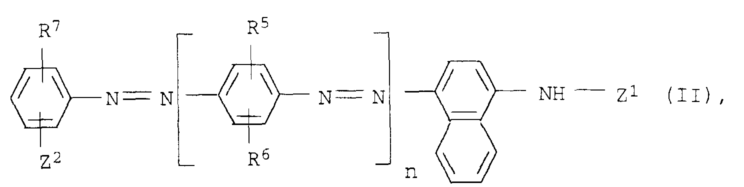 in der
- n: 0 oder 1 ist,
wobei, wenn n 0 ist,
- Z¹: C₁-C₁₅-Alkyl oder einen Rest der Formel L-NX¹X², worin L für C₂-C₈-Alkylen und X¹ und X² unabhängig voneinander jeweils für C₁-C₆-Alkyl oder zusammen mit dem sie verbindenden Stickstoffatom für einen 5- oder 6-gliedrigen gesättigten heterocyclischen Rest, der noch ein Sauerstoffatom im Ring enthalten kann, stehen,
- R⁷: Wasserstoff, C₁-C₁₅-Alkyl oder C₁-C₁₅-Alkoxy und
- Z²: einen Rest der Formel COOZ³, worin Z³ für C₁-C₁₅-Alkyl oder einen Rest der Formel L-NX¹X² steht, worin L, X¹ und X² jeweils die obengenannten Bedeutung besitzen, bedeuten,
mit der Maßgabe, daß wenn Z¹ für den Rest L-NX¹X² steht, die Summe der in den Resten X¹, X² und Z³ befindlichen Kohlenstoffatome mindestens 8 beträgt,
oder, wenn n 1 ist,
- Z¹: einen Rest der Formel L-NX¹X², worin L,X¹ und X² jeweils die obengenannte Bedeutung besitzen, und
- R⁵, R⁶, R⁷ und Z²: unabhängig voneinander jeweils Wasserstoff, C₁-C₁₅-Alkyl oder C₁-C₁₅-Alkoxy bedeuten,
mit der Maßgabe, daß jeweils in den Restepaaren R⁵/R⁶ und R⁷/Z² ein Rest von Wasserstoff verschieden ist.

Ein weiterer Gegenstand der vorliegenden Anmeldung sind Azofarbstoffe der Formel III 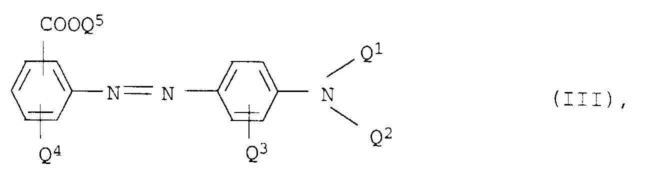 in der
- Q¹, Q² und Q³: unabhängig voneinander jeweils C₁-C₁₅-Alkyl,
- Q⁴: Wasserstoff, C₁-C₁₅-Alkyl oder C₁-C₁₅-Alkoxy und
- Q⁵: C₁-C₁₅-Alkyl oder einen Rest der Formel L-NX¹X² bedeuten, worin L für C₂-C₈-Alkylen und X¹ und X² unabhängig voneinander jeweils für C₁-C₆-Alkyl oder zusammen mit dem sie verbindenden Stickstoffatom für einen 5- oder 6-gliedrigen gesättigten heterocyclischen Rest, der noch ein Sauerstoffatom im Ring enthalten kann, stehen
mit der Maßgabe, daß die Summe der in den Resten Q¹, Q² und Q⁵ befindlichen Kohlenstoffatome mindestens 8 beträgt.

Bevorzugt sind Azofarbstoffe der Formel II, in der n 0 bedeutet.

Besonders bevorzugt sind Azofarbstoffe der Formel II, in der n 0 Z¹ C₁-C₁₃-Alkyl, R⁷ Wasserstoff und Z² den Rest COOZ³, worin Z³ die obengenannte Bedeutung besitzt, bedeuten.

Besonders bevorzugt sind weiterhin Azofarbstoffe der Formel III, in der Q¹ und Q² unabhängig voneinander jeweils C₁-C₁₃-Alkyl, Q³ Methyl, Q⁴ Wasserstoff und Q⁵ C₁-C₁₃-Alkyl bedeuten.

Die neuen Farbstoffe der Formeln II und III können nach an sich bekannten Methoden erhalten werden.

Zur Herstellung der Farbstoffe der Formel II kann man beispielsweise ein Amin der Formel IVa oder IVb 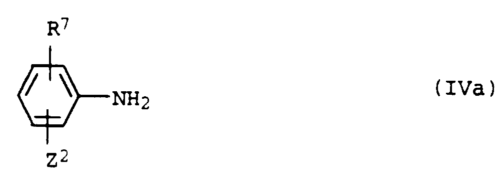 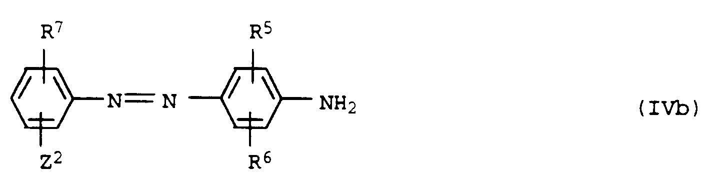 worin R⁵, R⁶, R⁷ und Z² jeweils die obengenannte Bedeutung besitzen, auf an sich bekanntem Wege diazotieren und mit einer Kupplungskomponente der Formel V 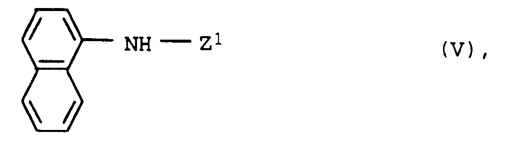 worin Z¹ die obengenannte Bedeutung besitzt, kuppeln.

Die Amine der Formel IVb wiederum sind zugänglich durch Diazotierung des Amins der Formel IVa und anschließendes Kuppeln mit einem Anilin der Formel IVc 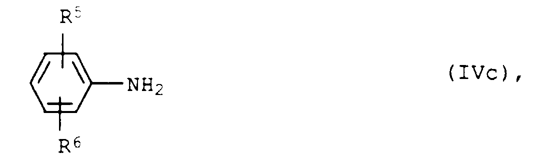 worin R⁵ und R⁶ jeweils die obengenannte Bedeutung besitzen.

Zur Herstellung der Farbstoffe der Formel III kann man beispielsweise ein Amin der Formel VI 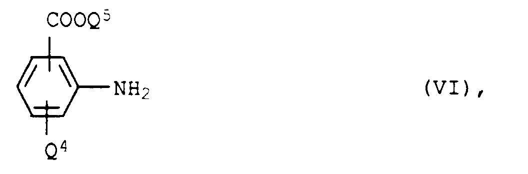 worin Q⁴ und Q⁵ jeweils die obengenannte Bedeutung besitzen, auf an sich bekanntem Weg diazotieren und mit einer Kupplungskomponente der Formel VII 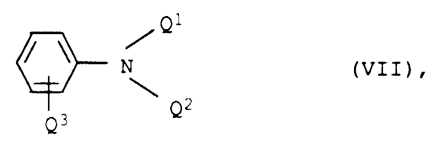 worin Q¹, Q² und Q³ jeweils die obengenannte Bedeutung besitzen, kuppeln.

Die neuen Azofarbstoffe der Formel II und III besitzen eine gute Löslichkeit in organischen Lösungsmitteln und eignen sich, wie oben bereits ausgeführt, in vorteilhafter Weise als pH-Wert-abhängige Markierungsmittel für Kohlenwasserstoffe.

Die folgenden Beispiele sollen die Erfindung näher erläutern.

### A) Herstellung

### Beispiel 1

107 g 4-(3'-Methylphenylazo)-3-methylanilin-hydrochlorid und 0,5 g eines anionisches Tensids wurden in einer Mischung aus 50 ml Wasser und 30 ml 5 N Salzsäure bei Raumtemperatur suspendiert. Nach Zugabe von 50 g Eis und 20 ml Toluol wurde mit einer konzentrierten wäßrigen Lösung von 6,9 g Natriumnitrit versetzt. Die Diazotierung war im Verlauf von 2 h bei 5 bis 10°C beendet, worauf der Nitritüberschuß mit Amidosulfonsäure entfernt wurde. Zur Lösung des Diazoniumsalzes tropfte man anschließend bei 10 bis 15°C eine Lösung von 29,5 g 1-(3-Diethylaminopropyl)aminonaphthalin in 35 ml Toluol. Anschließend wurde der pH-Wert des Reaktionsgemisches mit 2,5 N Natriumacetatlösung auf ca. 4 erhöht und die Kupplung war nach 30 min beendet. Nach Anheben des pH-Wertes mit 10 N-Natronlauge auf ca. 8 erfolgte eine Phasentrennung. Die organische Phase wurde dann bei 60°C durch mehrmaliges Extrahieren mit Wasser salzfrei gewaschen. Nach Abdestillieren des Toluols erhielt man 50 g eines ölartigen Farbstoffs der Formel 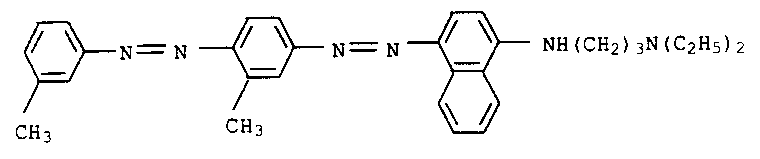 der sich mit roter Farbe gut in Aromaten löst. λₘₐₓ(Toluol) : 522 nm.

### Beispiel 2

Zu einer Mischung aus 33 ml 10 N Salzsäure und 10 ml Eisessig wurden bei 5 bis 10°C 35,2 g einer 58 gew.-%igen wäßrigen Lösung von Anthranilsäure(2-dimethylaminoethyl)ester getropft. Anschließend wurde bei 10 bis 15°C mit einer konzentrierten wäßrigen Lösung von 6,9 g Natriumnitrit versetzt, wobei der pH-Wert unter 0,5 gehalten wurde. Die Diazotierung war im Verlauf von 30 min bei 10 bis 15°C beendet, worauf der Nitritüberschuß mit Amidosulfonsäure entfernt wurde. Nach Zugabe von 1 g eines anionischen Tensids wurden zur Lösung des Diazoniumsalzes bei 10 bis 15°C innerhalb von 30 Minuten 26,2 g 1-(2-Ethylhexylamino)naphthalin, gelöst in 30 ml Eisessig, getropft. Anschließend wurde der Ansatz mit 100 ml Wasser verdünnt, und die Kupplung war nach 2 h beendet. Nach Zugabe von 100 ml Toluol und Anheben des pH-Wertes mit 10 N Natronlauge auf ca. 7 erfolgte eine Phasentrennung. Die organische Phase wurde dann bei 60°C durch mehrmaliges Extrahieren mit Wasser salzfrei gewaschen. Nach Abdestillieren des Toluols erhielt man 48 g eines ölartigen Farbstoffs der Formel 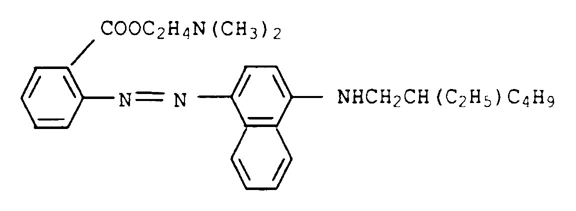 der sich mit oranger Farbe gut in Aromaten löst λₘₐₓ(Toluol): 448 nm.

### B) Anwendung

### Allgemeine Vorschrift

Handelsüblicher Dieselkraftstoff wird mit einer 40 gew.-%igen Lösung des Markierungsstoffs in einem handelsüblichen Gemisch höherer Aromaten - Shellsol® AB (Fa. Shell) - versetzt.

Der Zusatz an Markierungsmittel beträgt 10 ppm.
a) 50 ml markierter Dieselkraftstoff werden im Schütteltrichter mit 50 ml einer sauren Prüflösung, bestehend aus 45 g Wasser, 45 g Ethanol, 5 g p-Toluolsulfonsäure und 5 g Zinkchlorid, versetzt. Das Gemisch wird ca. 10 Sekunden lang kräftig geschüttelt. Nachdem sich die Phasen wieder getrennt haben, wird eine intensive Färbung der Prüflösungsphase erkennbar. Die wäßrige Phase kann gegen eine Lösung bekannter Konzentration photometrisch vermessen werden.
b) 20 ml markierter Dieselkraftstoff werden mit 20 ml Reagenzlösung (10 gew.-%ige Zinkchloridlösung in einem Wasser/Ethanol-Gemisch (60:40 v/v), pH-Wert durch Zugabe von 85 gew.-%iger Essigsäure auf 2 gestellt) kräftig geschüttelt. Dabei färbt sich die untere, wäßrige Phase deutlich erkennbar. Die wäßrige Phase kann gegen eine Lösung bekannter Konzentration photometrisch vermessen werden.

Die in den folgenden Tabellen aufgeführten Farbstoffe wurden nach Methode a) nachgewiesen. Sie können mit gleich gutem Erfolg auch nach Methode b) nachgewiesen werden.

In den folgenden Tabellen wurden die mit *) bezeichneten λₘₐₓ-Werte jeweils in Toluol und die mit **) bezeichneten λₘₐₓ-Werte jeweils in Wasser:Ethanol:p-Tolulsulfonsäure:Zinkchlorid 45:45:5:5 (w/w/w/w) gemessen.

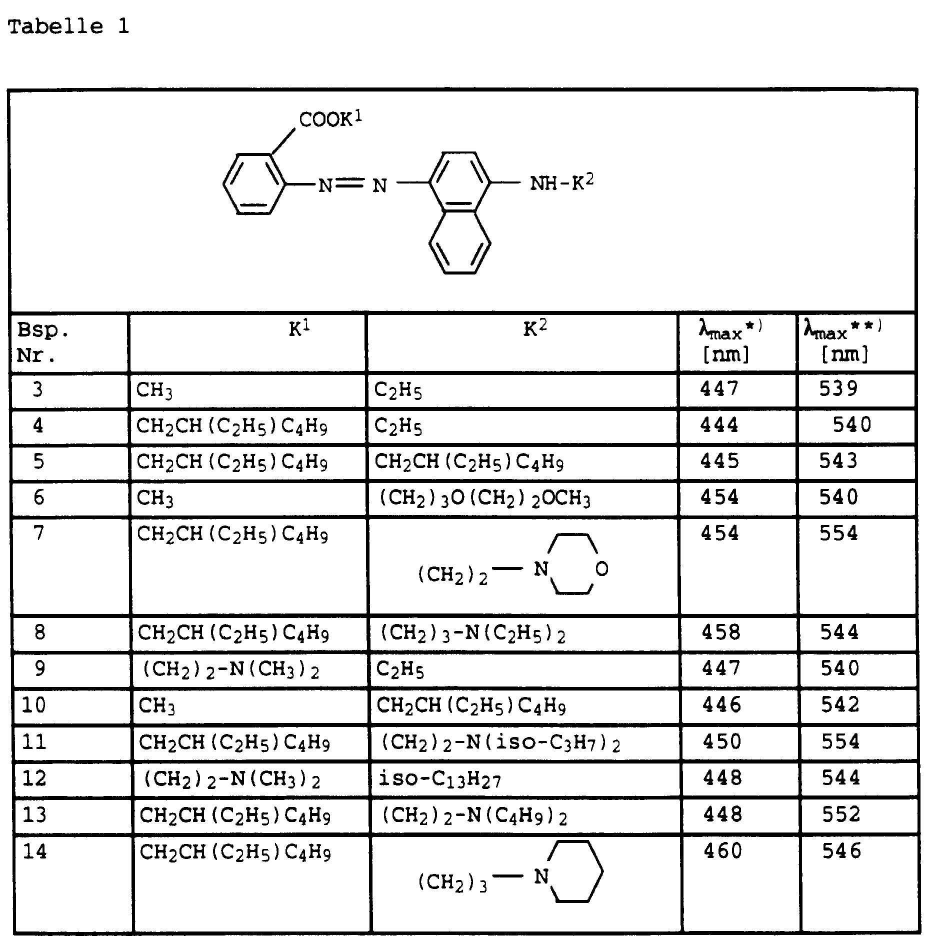

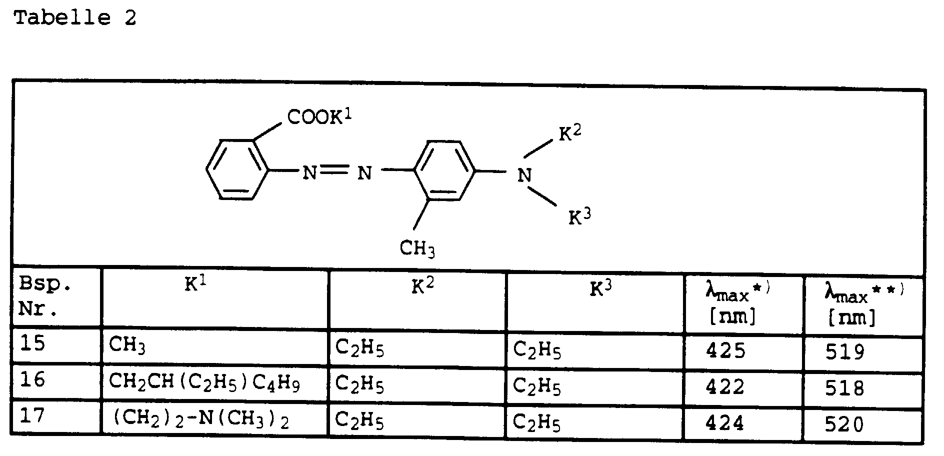

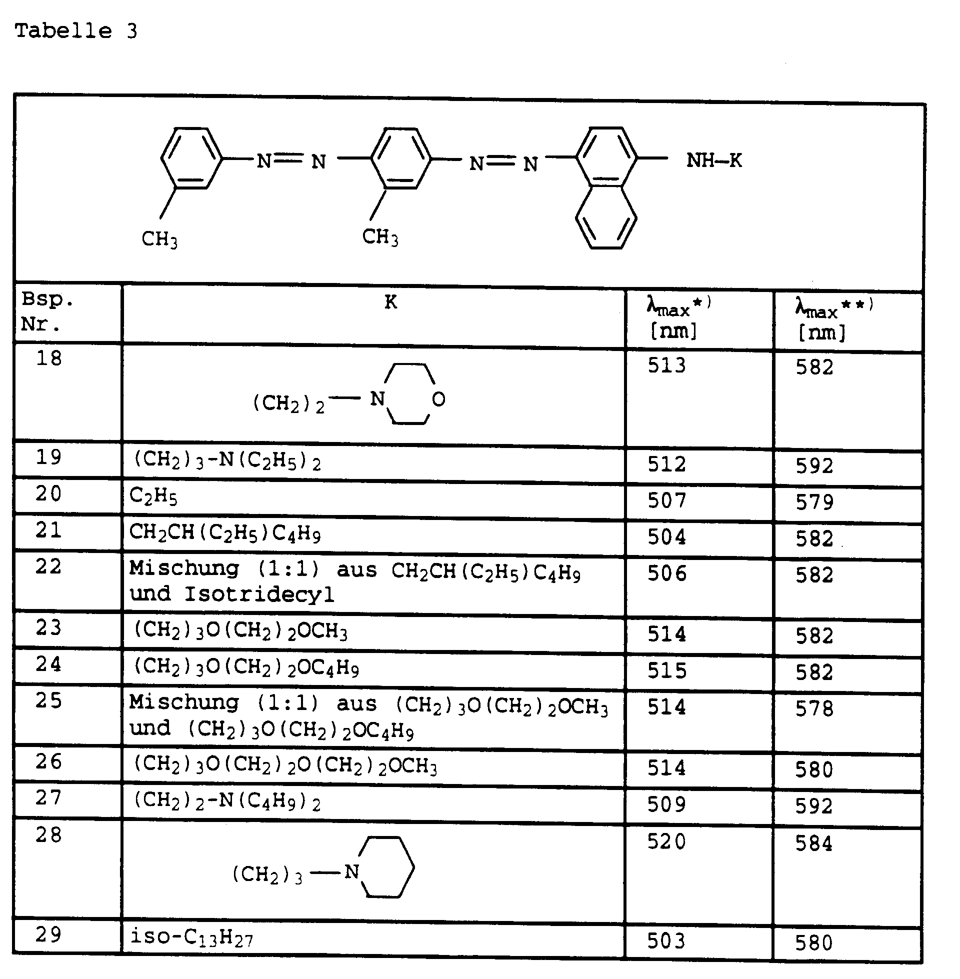

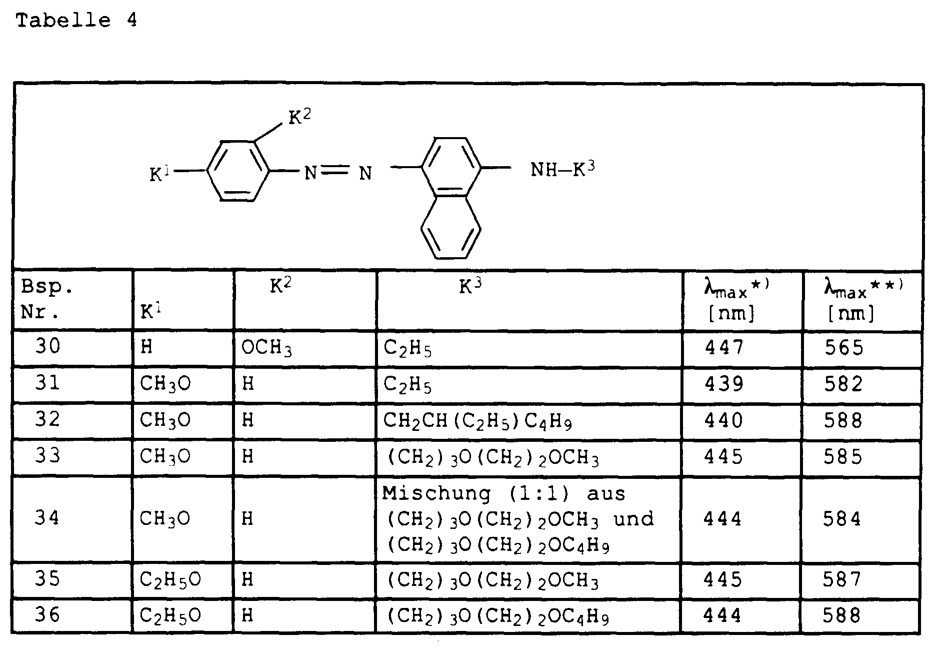

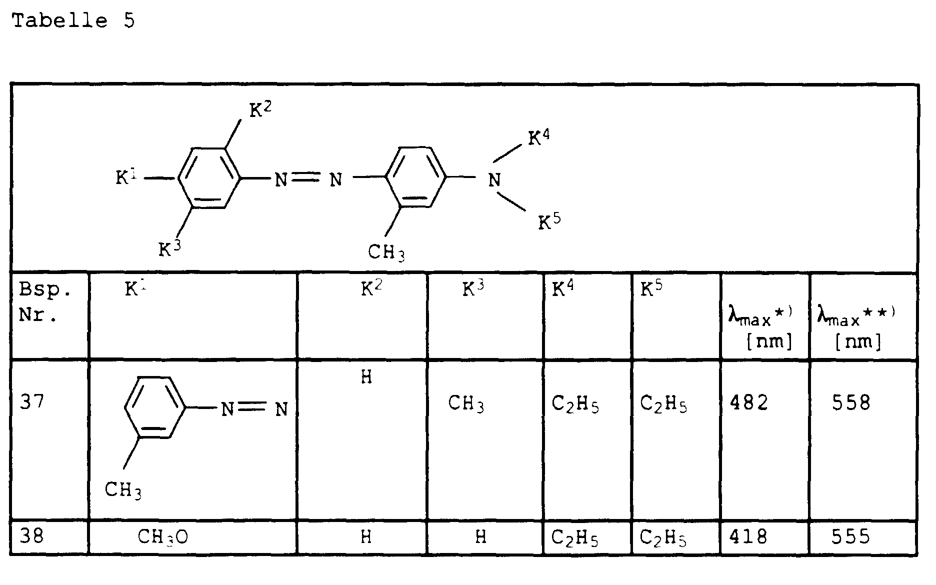

## Patentansprüche

1. Verwendung von Azofarbstoffen der Formel I 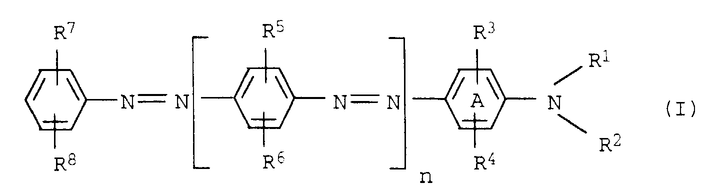 in der
der Ring A benzoanelliert sein kann,
n 0 oder 1,
R¹ Wasserstoff oder C₁-C₁₅-Alkyl, das durch 1 bis 4 Sauerstoffatome in Etherfunktion unterbrochen sein kann,
R² C₁-C₁₅-Alkyl, das durch 1 bis 4 Sauerstoffatome in Etherfunktion unterbrochen sein kann, oder einen Rest der Formel L-NX¹X², worin L für C₂-C₈-Alkylen und X¹ und X² unabhängig voneinander jeweils für C₁-C₆-Alkyl oder zusammen mit dem sie verbindenden Stickstoffatom für einen 5- oder 6-gliedrigen gesättigten heterocyclischen Rest, der noch ein Sauerstoffatom im Ring enthalten kann, stehen,
R³, R⁴, R⁵, R⁶ und R⁷ unabhängig voneinander jeweils Wasserstoff, C₁-C₁₅-Alkyl oder C₁-C₁₅-Alkoxy und
R⁸ Wasserstoff, C₁-C₁₅-Alkyl, C₁-C₁₅-Alkoxy, Cyano, Nitro oder einen Rest der Formel COOX³, worin X³ für Wasserstoff, C₁-C₁₅-Alkyl, das durch 1 bis 4 Sauerstoffatome in Etherfunktion unterbrochen sein kann, oder für einen Rest der Formel L-NX¹X² steht, worin L, X¹ und X² jeweils die obengenannten Bedeutung besitzen, bedeuten,
mit der Maßgabe, daß die Farbstoffe der Formel I keine Acetalgruppe im Molekül aufweisen,
als pH-Wert-abhängige Markierungsmittel für Kohlenwasserstoffe, wobei die Azofarbstoffe der Formel I in solcher Konzentration den Kohlenwasserstoffen zugesetzt werden, daß die Kohlenwasserstoffe dadurch für das menschliche Auge entweder überhaupt nicht oder nur wenig sichtbar angefärbt sind, sie jedoch unter Einwirkung einer Protonsäure, gegebenenfalls in Anwesenheit eines Halogenids der Metalle Zink, Aluminium oder Zinn, eine Farbreaktion ergeben.

2. Verwendung von Azofarbstoffen nach Anspruch 1, die der Formel Ia 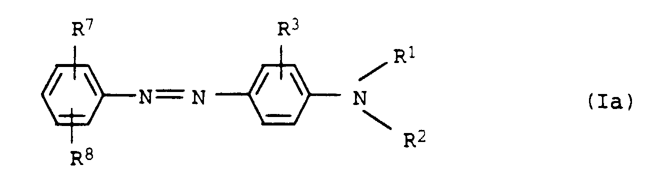 gehorchen, in der
R¹ Wasserstoff oder C₁-C₁₅-Alkyl, und
R² und R³ unabhängig voneinander jeweils C₁-C₁₅-Alkyl bedeuten und
R⁷ und R⁸ jeweils die in Anspruch 1 genannte Bedeutung besitzen.

3. Verwendung von Azofarbstoffen nach Anspruch 1, die der Formel Ib 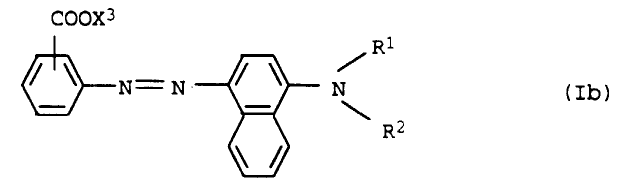 gehorchen, in der
R¹ Wasserstoff oder C₁-C₁₅-Alkyl und
R² C₁-C₁₅-Alkyl bedeuten und
X³ die in Anspruch 1 genannte Bedeutung besitzt.

4. Verwendung von Azofarbstoffen nach Anspruch 1, die der Formel Ic 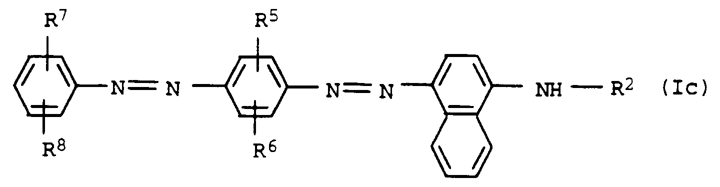 gehorchen, in der
R² C₁-C₁₅-Alkyl bedeutet und
R⁵, R⁶, R⁷ und R⁸ jeweils die in Anspruch 1 genannte Bedeutung besitzen.

5. Verfahren zum Nachweis der Anwesenheit von Azofarbstoffen der Formel I gemäß Anspruch 1 in Kohlenwasserstoffen, dadurch gekennzeichnet, daß man den Kohlenwasserstoff mit einem wäßrigalkoholischen oder alkoholischen Medium behandelt, das eine Protonsäure und gegebenenfalls ein Halogenid der Metalle Zink, Aluminium oder Zinn enthält.

6. Azofarbstoffe der Formel II 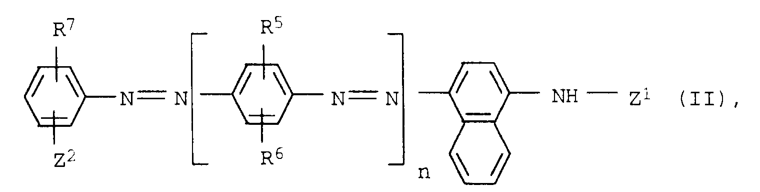 in der
n 0 oder 1,
wobei, wenn n 0 ist,
Z¹ C₁-C₁₅-Alkyl oder einen Rest der Formel L-NX¹X², worin L für C₂-C₈-Alkylen und X¹ und X² unabhängig voneinander jeweils für C₁-C₆-Alkyl oder zusammen mit dem sie verbindenden Stickstoffatom für einen 5- oder 6-gliedrigen gesättigten heterocyclischen Rest, der noch ein Sauerstoffatom im Ring enthalten kann, stehen,
R⁷ Wasserstoff, C₁-C₁₅-Alkyl oder C₁-C₁₅-Alkoxy und
Z² einen Rest der Formel COOZ³, worin Z³ für C₁-C₁₅-Alkyl oder einen Rest der Formel L-NX¹X² steht, worin L, X¹ und X² jeweils die obengenannten Bedeutung besitzen, bedeuten,
mit der Maßgabe, daß wenn Z¹ für den Rest L-NX¹X² steht, die Summe der in den Resten X¹, X² und Z³ befindlichen Kohlenstoffatome mindestens 8 beträgt,
oder, wenn n 1 ist,
Z¹ einen Rest der Formel L-NX¹X², worin L,X¹ und X² jeweils die obengenannte Bedeutung besitzen, und
R⁵, R⁶, R⁷ und Z² unabhängig voneinander jeweils Wasserstoff, C₁-C₁₅-Alkyl oder C₁-C₁₅-Alkoxy bedeuten,
mit der Maßgabe, daß jeweils in den Restepaaren R⁵/R⁶ und R⁷/Z² ein Rest von Wasserstoff verschieden ist.

7. Azofarbstoffe nach Anspruch 6, dadurch gekennzeichnet, daß n 0 bedeutet.

8. Azofarbstoffe der Formel III 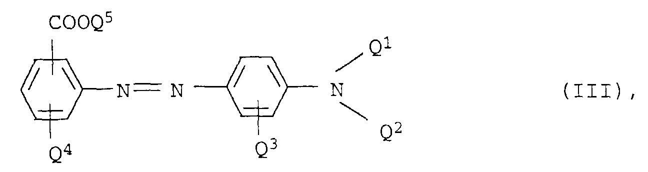 in der
Q¹, Q² und Q³ unabhängig voneinander jeweils C₁-C₁₅-Alkyl,
Q⁴ Wasserstoff, C₁-C₁₅-Alkyl oder C₁-C₁₅-Alkoxy und
Q⁵ C₁-C₁₅-Alkyl oder einen Rest der Formel L-NX¹X² bedeuten, worin L für C₂-C₈-Alkylen und X¹ und X² unabhängig voneinander jeweils für C₁-C₆-Alkyl oder zusammen mit dem sie verbindenden Stickstoffatom für einen 5- oder 6-gliedrigen gesättigten heterocyclischen Rest, der noch ein Sauerstoffatom im Ring enthalten kann, stehen
mit der Maßgabe, daß die Summe der in den Resten Q¹, Q² und Q⁵ befindlichen Kohlenstoffatome mindestens 8 beträgt.

## Claims

1. Use of an azo dye of the formula I 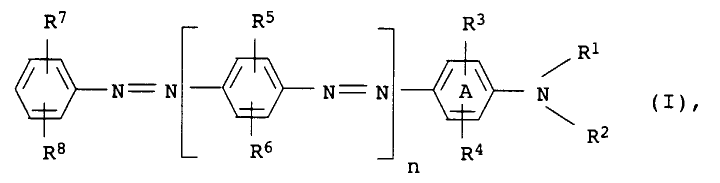 where
the ring A may be benzofused,
n is 0 or 1,
R¹ is hydrogen or C₁-C₁₅-alkyl which may be interrupted by from 1 to 4 ether oxygen atoms,
R² is C₁-C₁₅-alkyl which may be interrupted by from 1 to 4 ether oxygen atoms, or a radical of the formula L-NX¹X², where L is C₂-C₈-alkylene and X¹ and X² independently of one another are each C₁-C₆-alkyl or, together with the nitrogen atom linking them, form a 5-membered or 6-membered saturated heterocyclic radical which may furthermore contain an oxygen atom in the ring,
R³, R⁴, R⁵, R⁶ and R⁷ independently of one another are each hydrogen, C₁-C₁₅-alkyl or C₁-C₁₅-alkoxy and
R⁸ is hydrogen, C₁-C₁₅-alkyl, C₁-C₁₅-alkoxy, cyano, nitro or a radical of the formula COOX³, where X³ is hydrogen, C₁-C₁₅-alkyl which may be interrupted by from I to 4 ether oxygen atoms, or is a radical of the formula L-NX¹X², where L, X¹ and X² each have the abovementioned meanings,
with the proviso that the dye of the formula I does not have an acetal group in the molecule,
as a pH-dependent marker for hydrocarbons, the azo dye of the formula I being added to the hydrocarbons in a concentration such that the hydrocarbons thereby appear to the human eye to have either no color at all or only a slight color, but, under the action of a protic acid, in the presence or absence of a halide of the metals zinc, aluminum or tin, they give a color reaction.

2. Use of an azo dye as claimed in claim 1, which is of the formula Ia 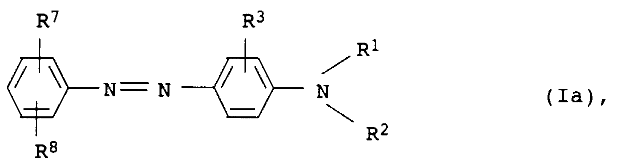 where
R¹ is hydrogen or C₁-C₁₅-alkyl,
R² and R³ independently of one another are each C₁-C₁₅-alkyl and
R⁷ and R⁸ each have the meanings stated in claim 1.

3. Use of an azo dye as claimed in claim 1, which is of the formula Ib 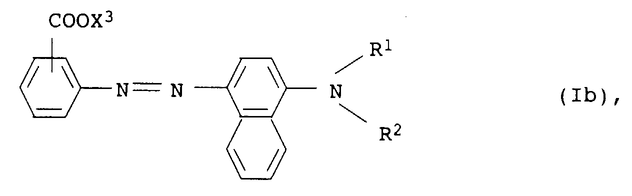 where
R¹ is hydrogen or C₁-C₁₅-alkyl,
R² is C₁-C₁₅-alkyl and
X³ has the meanings stated in claim 1.

4. Use of an azo dye as claimed in claim 1, which is of the formula Ic 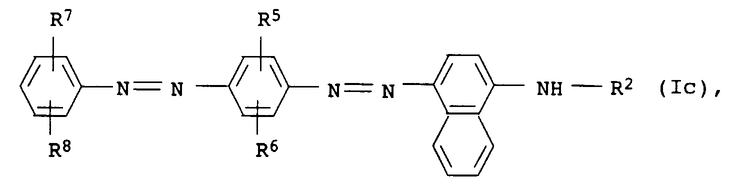 where
R² is C₁-C₁₅-alkyl and
R⁵, R⁶, R⁷ and R⁸ each have the meanings stated in claim 1.

5. A method for detecting the presence of azo dyes of the formula I as claimed in claim 1 in hydrocarbons, wherein the hydrocarbon is treated with an aqueous alcoholic or alcoholic medium which contains a protic acid and, if required, a halide of the metals zinc, aluminum or tin.

6. An azo dye of the formula II 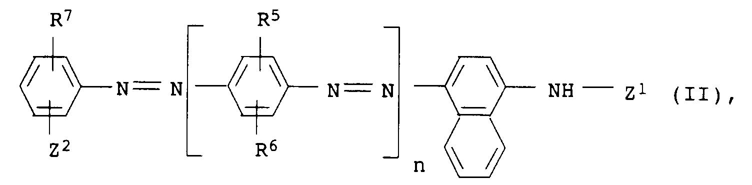 where
n is 0 or 1
and, when n is 0,
Z¹ is C₁-C₁₅-alkyl or is a radical of the formula L-NX¹X², where L is C₂-C₈-alkylene and X¹ and X² independently of one another are each C₁-C₆-alkyl or, together with the nitrogen atom linking them, form a 5-membered or 6-membered saturated heterocyclic radical which may furthermore contain an oxygen atom in the ring,
R⁷ is hydrogen, C₁-C₁₅-alkyl or C₁-C₁₅-alkoxy and
Z² is a radical of the formula COOZ³, where Z³ is C₁-C₁₅-alkyl or is a radical of the formula L-NX¹X², where L, X¹ and X² each have the abovementioned meanings,
with the proviso that, when Z¹ is L-NX¹X², the sum of the carbon atoms present in the radicals X¹, X² and Z³ is at least 8,
or, when n is 1,
Z¹ is a radical of the formula L-NX¹X², where L, X¹ and X² each have the abovementioned meanings, and
R⁵, R⁶, R⁷ and Z² independently of one another are each hydrogen, C₁-C₁₅-alkyl or C₁-C₁₅-alkoxy,
with the proviso that, in each of the pairs of radicals R⁵/R⁶ and R⁷/Z², one radical is not hydrogen.

7. An azo dye as claimed in claim 6, wherein n is 0.

8. An azo dye of the formula III 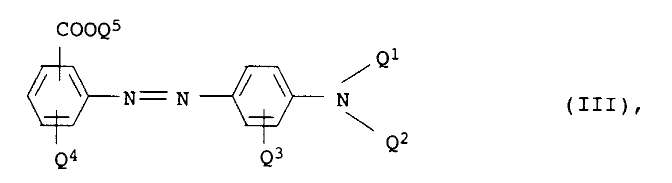 where
Q¹, Q² and Q³ independently of one another are each C₁-C₁₅-alkyl,
Q⁴ is hydrogen, C₁-C₁₅-alkyl or C₁-C₁₅-alkoxy and
Q⁵ is C₁-C₁₅-alkyl or is a radical of the formula L-NX¹X², where L is C₂-C₈-alkylene and X¹ and X² independently of one another are each C₁-C₆-alkyl or, together with the nitrogen atom linking them, form a 5-membered or 6-membered saturated heterocyclic radical which may furthermore contain an oxygen atom in the ring,
with the proviso that the sum of the carbon atoms present in the radicals Q¹, Q² and Q⁵ is at least 8.

## Revendications

1. Utilisation de colorants azoïques de formule I 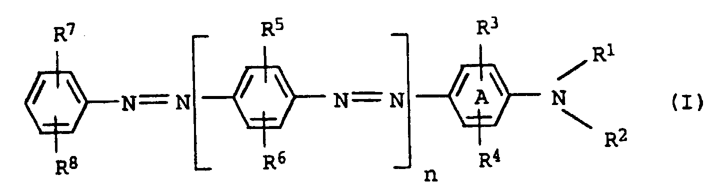 dans laquelle
le noyau A peut être benzocondensé,
n vaut 0 ou 1,
R¹ est un atome d'hydrogène ou un groupe alkyle en C₁-C₁₅, qui peut être interrompu par 1 à 4 atomes d'oxygène en fonction éther,
R² est un groupe alkyle en C₁-C₁₅, qui peut être interrompu par 1 à 4 atomes d'oxygène en fonction éther, ou encore un radical de formule L-NX¹X² dans laquelle L est un groupe alkylène en C₂-C₈ et X¹ et X² représentent chacun indépendamment de l'autre un groupe alkyle en C₁-C₆ ou forment avec l'atome d'azote qui les relie un radical hétérocyclique saturé à 5 ou 6 chaînons, qui peut encore contenir un atome d'oxygène dans le noyau,
R³, R⁴, R⁵, R⁶ et R⁷ représentent chacun indépendamment des autres un atome d'hydrogène ou un groupe alkyle en C₁-C₁₅ ou alcoxy en C₁-C₁₅, et
R⁸ est un atome d'hydrogène ou un radical alkyle en C₁-C₁₅, alcoxy en C₁-C₁₅, cyano, nitro, ou un radical de formule COOX³ dans laquelle X³ est mis pour un atome d'hydrogène, pour un groupe alkyle en C₁-C₁₅ pouvant être interrompu par 1 à 4 atomes d'oxygène en fonction éther, ou encore pour un radical de formule L-NX¹X² dans laquelle L, X¹ et X² ont chacun les significations données ci-dessus,
à la condition que les colorants de formule I ne comportent pas de groupe acétal dans leur molécule,
en tant qu'agent de marquage, dépendant du pH, pour hydrocarbures, les colorants azoïques de formule I étant ajoutés aux hydrocarbures en une concentration telle que les hydrocarbures en subissent une coloration qui ou bien n'est absolument pas visible pour l'oeil humain, ou n'est que peu visible, mais, sous l'action d'un acide protonique, éventuellement en présence d'un halogénure des métaux zinc, aluminium ou étain, donnent une réaction colorée.

2. Utilisation de colorants azoïques selon la revendication 1, ayant la formule la 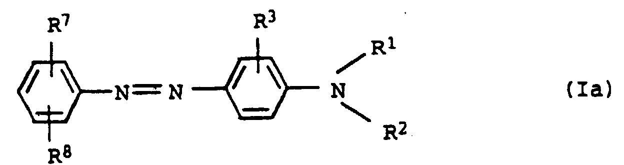 dans laquelle
R¹ est un atome d'hydrogène ou un groupe alkyle en C₁-C₁₅, et
R² et R³ représentent chacun indépendamment de l'autre un groupe alkyle en C₁-C₁₅, et
R⁷ et R⁸ ont chacun les significations données dans la revendication 1.

3. Utilisation de colorants azoïques selon la revendication 1, ayant la formule Ib 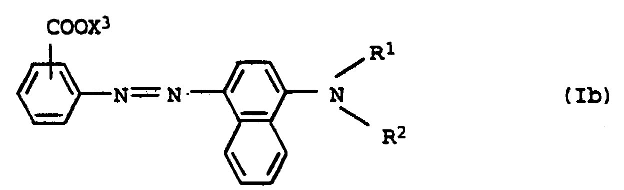 dans laquelle
R¹ est un atome d'hydrogène ou un groupe alkyle en C1-C15, et
R² est un groupe alkyle en C₁-C₁₅, et
X³ a les significations données dans la revendication 1.

4. Utilisation de colorants azoïques selon la revendication 1, ayant la formule Ic 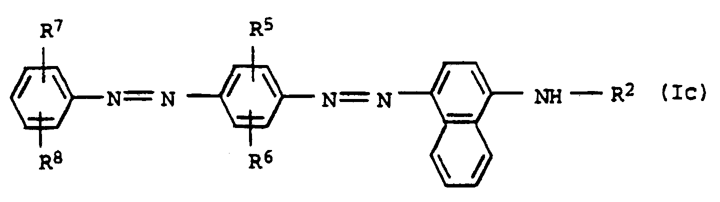 dans laquelle
R² est un groupe alkyle en C₁-C₁₅, et
R⁵, R⁶, R⁷ et R⁸ ont chacun les significations données dans la revendication 1.

5. Procédé pour détecter la présence de colorants azoïques de formule I selon la revendication 1 dans des hydrocarbures, caractérisé en ce qu'on traite l'hydrocarbure avec un milieu aqueux-alcoolique ou alcoolique qui contient un acide protonique et éventuellement un halogénure des métaux zinc, aluminium ou étain.

6. Colorants azoïques de formule II 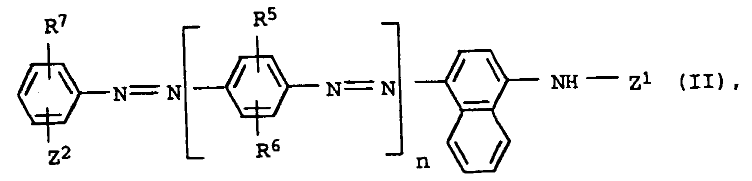 dans laquelle
n vaut 0 ou 1,
auquel cas, quand n vaut 0,
Z¹ est un groupe alkyle en C₁-C₁₅ ou un radical de formule L-NX¹X² dans laquelle L est un groupe alkylène en C₂-C₈, et X¹ et X² représentent chacun indépendamment de l'autre un groupe alkyle en C₁-C₆ ou encore, avec l'atome d'azote qui les relie, forment un radical hétérocyclique saturé à 5 ou 6 chaînons, qui peut encore contenir un atome d'oxygène dans le noyau,
R⁷ est un atome d'hydrogène ou un groupe alkyle en C₁-C₁₅ ou alcoxy en C₁-C₁₅, et
Z² est un radical de formule COOZ³ dans laquelle Z³ est un groupe alkyle en C₁-C₁₅ ou un radical de formule L-NX¹X² dans laquelle L, X¹ et X² ont chacun les significations données ci-dessus,
à la condition que, quand Z¹ est le radical L-NX¹X², le nombre total d'atomes de carbone se trouvant dans les radicaux X¹, X² et Z³ soit d'au moins 8,
ou encore, quand n vaut 1
Z¹ est un radical de formule L-NX¹X² dans laquelle L, X¹ et X² ont chacun les significations données ci-dessus, et
R⁵, R⁶, R⁷ et Z² représentent chacun indépendamment des autres un atome d'hydrogène ou un groupe alkyle en C₁-C₁₅ ou alcoxy en C₁-C₁₅,
à la condition que, dans chacune des paires de radicaux R⁵/R⁶ et R⁷/Z², l'un des radicaux ne soit pas un atome d'hydrogène.

7. Colorants azoïques selon la revendication 6, caractérisés en ce que n vaut 0.

8. Colorants azoïques de formule III 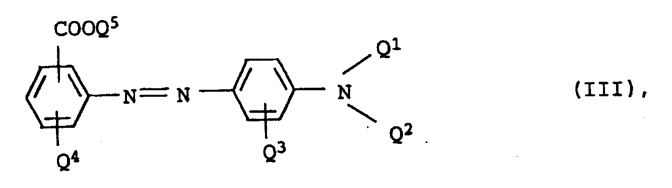 dans laquelle
Q¹, Q² et Q³ représentent chacun indépendamment des autres un groupe alkyle en C₁-C₁₅,
Q⁴ est un atome d'hydrogène ou un groupe alkyle en C₁-C₁₅ ou alcoxy en C₁-C₁₅, et
Q⁵ est un groupe alkyle en C₁-C₁₅ ou un radical de formule L-NX¹X², dans laquelle L est un groupe alkylène en C₂-C₈, et X¹ et X² représentent chacun indépendamment de l'autre un groupe alkyle en C₁-C₆ ou encore, avec l'atome d'azote qui les relie, forment un radical hétérocyclique saturé à 5 ou 6 chaînons, qui peut encore contenir un atome d'oxygène dans le noyau,
à la condition que le nombre total d'atomes de carbone se trouvant dans les radicaux Q¹, Q² et Q⁵ soit d'au moins 8.
